# EUROPEAN PATENT SPECIFICATION

(11) **EP 4 032 536 B1**
(45) Date of publication and mention of the grant of the patent: **07.10.2026**
(21) Application number: 21382060.8
(22) Date of filing: 26.01.2021
(51) Int. Cl.: A61K 35/745, A61K 35/744, A61P 1/00, A61P 37/08

(54) **PROBIOTIC COMPOSITION FOR INFANTS COMPRISING LACTOBACILLUS SALIVARIUS CECT9527 AND BIFIDOBACTERIUM LONGUM CECT30257 AND ITS USES**
PROBIOTISCHE ZUSAMMENSETZUNG FÜR KLEINKINDER UMFASSEND LACTOBACILLUS SALIVARIUS CECT9527 AND BIFIDOBACTERIUM LONGUM CECT30257 UND DEREN VERWENDUNGEN
COMPOSITION PROBIOTIQUE POUR NOURRISSONS COMPRENANT LACTOBACILLUS SALIVARIUS CECT9527 AND BIFIDOBACTERIUM LONGUM CECT30257 ET SES UTILISATIONS

(43) Date of publication of application: 27.07.2022
(73) Proprietor: Probisearch, S.L.U., 28760 Tres Cantos, Madrid (ES)
(72) Inventor: JIMÉNEZ QUINTANA, Esther, 28760 Tres Cantos (Madrid) (ES)
(74) Representative: Pons IP

(56) References cited:
- EP-A1- 2 308 498
- EP-A1- 3 412 766
- EP-B1- 2 768 311
- WO-A1-2015/086788
- WO-A1-2017/043962
- WO-A1-2019/019961
- IE-A1- 990 033
- US-A1- 2001 036 453
- US-B2- 8 470 579
- HAJELA NEERJA ET AL: "Gut microbiome, gut function, and probiotics: Implications for health", INDIAN JOURNAL OF GASTROENTEROLOGY, INDIAN SOCIETY OF GASTROENTEROLOGY, BOMBAY, IN, vol. 34, no. 2, 1 March 2015 (2015-03-01), pages 93 - 107, XP037125642, ISSN: 0254-8860, [retrieved on 20150429], DOI: 10.1007/S12664-015-0547-6
- YAYOI SATOH ET AL: "Bifidobacteria prevents necrotizing enterocolitis and infection in preterm infants", INTERNATIONAL JOURNAL OF PROBIOTICS & PREBIOTICS, vol. 2, no. 2/3, 1 January 2007 (2007-01-01), pages 149 - 154, XP055536434, ISSN: 1555-1431
- DATABASE UniProt [online] 15 January 2021 (2021-01-15), "Proteomes - Lactobacillus salivarius (strain CECT 5713)", XP002803529, Database accession no. UP000001090

## Description

The present invention relates to probiotic compositions comprising *Lactobacillus salivarius and Bifidobacterium longum* for use in the reduction of pathogenic microorganisms in the intestinal tract, in the reduction of proinflammatory response, in the treatment and/or prevention of necrotizing enterocolitis, or in the treatment and/or prevention of allergies in a subject, wherein
- the *L. salivarius* strain *is L. salivarius* CECT9527 or a strain derived thereof or a strain having at least 95% of identity with the 16S rRNA sequence of the *L. salivarius* CECT9527, and
- the *B. longum strains is B. longum* CECT30257 or a strain derived thereof or a strain having at least 95% of identity with the 16S rRNA sequence of the B. *longum* CECT30257. Therefore, the present invention belongs to the technical field of food, in particular, food with therapeutic properties due to the presence of beneficial microorganisms for the health.

### BACKGROUND ART

Despite the latest clinical advances in the treatment of preterm infants, low-birth weight (LBW) infants and term infants, problems like infections and inflammation, especially intestinal inflammation, are still a significant cause of morbidity and mortality, especially in small preterm neonates.

According to the Worth Health Organization, infections including sepsis, pneumonia, tetanus and diarrhea, account for about 36% of the 3.3 million neonatal deaths worldwide per year. The diagnosis of neonatal sepsis is complicated by the frequent presence of noninfectious conditions that resemble sepsis, especially in preterm infants, and by the absence of optimal diagnostic tests. Since neonatal sepsis is a high-risk disease, especially in preterm infants, clinicians are compelled to empirically administer antibiotics to infants with risk factors and/or signs of suspected sepsis. Unfortunately, both broad-spectrum antibiotics and prolonged treatment with empirical antibiotics are associated with adverse outcomes and increase antimicrobial resistance rates.

Infants, particularly preterm and LBW infants can also suffer from immature intestinal vascularization and, consequently, reduced blood flow to the intestinal mucosa. Impaired blood flow can delay intestinal development, impair nutrient absorption and tolerance to enteral feeds, alter intestinal motility and it is believed to be a causative factor in the pathogenesis of necrotizing enterocolitis (NEC). NEC affects mainly preterm infants. It is the most common surgical emergency in newborns. Around 12% of premature infants weighing less than 1500 g become afflicted with NEC. Mortality ranges from 20% to 50% and morbidity includes strictures, adhesions, and short bowel syndrome.

Given the high incidence and mortality/morbidity of sepsis and NEC in preterm infants and its long-term consequences on growth and development, efforts to reduce the rates of infection and inflammatory response in this vulnerable population are one of the most important interventions in neonatal care.

Due to its importance several inventions have tried to address either one or both problems through the use of probiotics or probiotic compositions to help stimulate the development of healthy intestinal flora and to reduce the risk of intestinal infections or inflammations. The document US20010036453A1 discloses probiotic microbes and compositions for the treatment and prevention of infections originating from the intestine. The document WO2017043962A1 provides a composition of probiotics for stimulating microbiota health after non-natural birth of infants. The document EP2768311B1 discloses a synthetic nutritional composition for use in the promotion of intestinal angiogenesis and of nutrient absorption.

Despite the aforementioned works, alternative probiotic compositions are required for the reduction of infections and inflammation in infants, especially preterm infants and/or LBW infants.

### DETAILED DESCRIPTION OF THE INVENTION

The invention is set out in the appended set of claims.

The present invention discloses a probiotic composition comprising *Lactobacillus salivarius* and *Bifidobacterium longum* bacteria, specifically, *L. salivarius* CECT9527 or a strain derived thereof or a strain having at least 95% of identity with the 16S rRNA sequence of the *L. salivarius* CECT9527, and B. *longum* CECT30257 or a strain derived thereof or a strain having at least 95% of identity with the 16S rRNA sequence of the *B. longum* CECT30257, which, surprisingly, has antagonistic properties against pathogenic bacteria in the intestinal tract of infants, especially preterm infants. In a clinical study, the inventors noticed that the aforementioned composition shown several advantages in the patient treat group in comparison with the placebo control group, namely:
- Reduced number of days of stay at the Neonatal Unit;
- Reduced number of adverse events (e.g. gastrointestinal disorders, sepsis);
- Early colonization of the intestinal tract by *Lactobacillus* and *Bifidobacterium*;
- Reduction of inflammation markers;
- Increase of immunomodulatory infection response markers.

All these advantages turn the probiotic composition here disclosed into a useful tool for the reduction of pathogenic strains in the gastrointestinal tract, the reduction of proinflammatory response, the treatment and/or prevention of necrotic enterocolitis, and/or the treatment and/or prevention of allergies in a subject.

Therefore, a first aspect of the present invention relates to a probiotic composition comprising *L. salivarius* CECT9527 or a strain derived thereof or a strain having at least 95% of identity with the 16S rRNA sequence of the *L. salivarius* CECT9527, and *B. longum* CECT30257 or a strain derived thereof or a strain having at least 95% of identity with the 16S rRNA sequence of the B. *longum* CECT30257, from here onwards the "probiotic composition of the invention", for use in the reduction of pathogenic strains in the gastrointestinal tract, in the reduction of proinflammatory response, in the treatment and/or prevention of necrotic enterocolitis, or in the treatment and/or prevention of allergies in a subject, from here onwards "use of the invention".

The term "probiotic" as used herein refers to a microbial cell preparation or microbial cell component that has a beneficial effect on the health or well-being of the host. Said benefits may be obtained both when the microbial cell preparation comprises viable cells or unviable cells, wherein the cells have been inactivated or dead, or even be present as fragments such as DNA materials and cell wall components. Therefore, in a preferred embodiment of the use of the invention, the probiotic composition comprises viable and/or unviable cells of *L. salivarius* CECT9527, or a strain derived thereof or a strain having at least 95% of identity with the 16S rRNA sequence of the *L. salivarius* CECT9527, and B. *longum* CECT30257 or a strain derived thereof or a strain having at least 95% of identity with the 16S rRNA sequence of the B. *longum* CECT30257.

In the present invention, the probiotic composition comprises cells from the above strains of *L. salivarius* and B. *longum.*

*L. salivarius* is a rod-shaped, Gram-positive species of lactic acid bacterium that has antimicrobial activity due to the production of bacteriocins. The scientific classification of *L. salivarius* is: Kingdom: *Bacteria*; Division: *Firmicute*; Class: *Bacilli*; Order: *Lactobacillales*; Family: *Lactobacillaceae*, Gender: *Lactobacillus*; Species: *L*. *salivarius.* In the use of the invention, *L. salivarius* is the strain *L. salivarius* CECT9527 or a strain derived thereof or a strain having at least 95% of identity with the 16S rRNA sequence of the *L. salivarius* CECT9527.

*L. salivarius* strain was isolated from the vagina of a healthy women and deposited by Probisearch, S.L.U., Calle Santiago Grisolia 2, Tres Cantos, Spain, in accordance with the Budapest Treaty on the International Recognition of the Deposit of Microorganisms for the Purpose of Patent Procedure at the Colección Española de Cultivos Tipo (Edificio 3 CUE, Parc Cientific Universitat de Valencia, Catedrático Agustin Escardino, 9, 46980 Paterna (Valencia) SPAIN) on the 13th December 2017. It was assigned the Accession Number CECT9527 (hereinafter also named *L*. *salivarius* CECT9527).

Herein, also provided for those microorganisms or bacteria strains derived from *L*. *salivarius* CECT9527 and that retain the ability to reduce the pathogenic strains in the gastrointestinal tract, to reduce the proinflammatory response, to be use in the treatment and/or prevention of necrotic enterocolitis, and to be used in the treatment and/or prevention of allergies in a subject. Examples of strains derived from *L*. *salivarius* CECT9527 may be mutants exhibiting changes in their genome compared to the genome of *L. salivarius* CECT9527, wherein said changes do not affect the above-mentioned abilities. Strains derived from *L. salivarius* CECT9527 can be formed naturally or intentionally by mutagenesis methods known to those skilled in the art, such as, but not limited to, the growth of the parental strain in the presence of mutagenic agents or stressors or genetically engineered to modify, delete and / or insert specific genes. An example of assay for testing if a strain derived from *L*. *salivarius* CECT9527 has the properties of the parent strain *L. salivarius* CECT9527 is provided in the Examples of the present application.

In addition, the present invention also relates to microorganisms or bacteria strains having at least 95% of identity with the 16S rRNA sequence (SEQ ID NO: 1) of the *L*. *salivarius* CECT9527 (Stackebrandt and Goebel. 1994. Int. J. Syst. Bacteriol. 44: 846-849). In a more preferred embodiment, *L. salivarius is* a strain having at least 96, 97, 98, or 99% of identity with the 16S rRNA sequence of the *L. salivarius* CECT9527.

*Bifidobacterium longum* is a Gram negative bacteria, catalase-negative, rounded in shape, located in the gastrointestinal tract where it produces lactic acid. The scientific classification of *Bifidobacterium longum* is: Kingdom: *Bacteria*; Division: *Firmicutes*; Class: *Actinobacteria*; Order: *Bifidobacteriales*; Family: *Bifidobacteriaceae*; Genus: *Bifidobacterium*; Species: *Bifidobacterium longum.* In the use of the invention, B. *longum* is the strain B. *longum* CECT30257 or a strain derived thereof or a strain having at least 95% of identity with the 16S rRNA sequence of the B. *longum* CECT30257.

*B. longum* strain was isolated from human milk of healthy women and deposited by Probisearch, S.L.U, Calle Santiago Grisolia 2, Tres Cantos, Spain, in accordance with the Budapest Treaty on the International Recognition of the Deposit of Microorganisms for the Purpose of Patent Procedure at the Colección Española de Cultivos Tipo (Edificio 3 CUE, Parc Cientific Universitat de Valencia, Catedrático Agustín Escardino, 9, 46980 Paterna (Valencia) SPAIN ) on the 2^{nd} December 2020.

It was assigned the Accession Number CECT30257 (hereinafter also named B. *longum* CECT30257).

Herein, also provided for those microorganisms or bacteria strains derived from B. *longum* CECT30257 and that retain the ability to reduce the pathogenic strains in the gastrointestinal tract, to reduce the proinflammatory response, to be use in the treatment and/or prevention of necrotic enterocolitis, and to be used in the treatment and/or prevention of allergies in a subject. Examples of strains derived from B. *longum* CECT30257 may be mutants exhibiting changes in their genome compared to the genome of B. *longum* CECT30257, wherein said changes do not affect the above-mentioned abilities. Strains derived from B. *longum* CECT30257 can be formed naturally or intentionally by mutagenesis methods known to those skilled in the art, such as, but not limited to, the growth of the parental strain in the presence of mutagenic agents or stressors or genetically engineered to modify, delete and / or insert specific genes. An example of assay for testing if a strain derived from B. *longum* CECT30257 has the properties of the parent strain B. *longum* CECT30257 is provided in the Examples of the present application.

In addition, the present invention also relates to microorganisms or bacteria strains having at least 95% of identity with the 16S rRNA sequence (SEQ ID NO: 2) of the B. *longum* CECT30257 (Stackebrandt and Goebel. 1994. Cited *ad supra*). In a more preferred embodiment, *B. longum is* a strain having at least 96, 97, 98, or 99% of identity with the 16S rRNA sequence of the *B. longum* CECT30257.

In the present invention, "identity" or "sequence identity" is understood to mean the degree of similarity between two nucleotides (or amino acid sequences) obtained by aligning the two sequences. Depending on the number of common residues between the aligned sequences, a different degree of identity, expressed as a percentage, will be obtained. The degree of identity between two nucleotide sequences may be determined by conventional methods, for example, by standard sequence alignment algorithms known in the state of the art, such as, for example, BLAST [Altschul S.F. et al. Basic local alignment search tool. J Mol Biol. 1990 Oct 5; 215(3): 403-10]. The BLAST programs, for example, BLASTN, BLASTX, and T BLASTX, BLASTP and TBLASTN, are in the public domain at The National Center for Biotechonology Information (NCBI) website. The skilled person in the art understands that mutations in the nucleotide sequence of genes that lead to conservative amino acid substitutions at non-critical positions might be evolutionarily neutral mutations which do not affect its global structure or its functionality.

*L. salivarius* and B. *longum,* or their preferred embodiments previously mentioned, may each be present in the probiotic composition of the invention in any therapeutically effective amount. In the present invention "therapeutically effective amount" is that amount of each microorganism that, when administered to a subject, is high enough to significantly positively modify the condition to be treated, i.e. to produce the desired effect, such as the reduction of pathogenic strains in the gastrointestinal tract, the reduction of proinflammatory response, the treatment and/or prevention of necrotic enterocolitis, and the treatment and/or prevention of allergies in a subject, but low enough to avoid serious side effects (at reasonable benefit/risk ratio) within the scope of sound medical judgment. As the skilled person in art knows, the therapeutically effective amount of the microorganisms will vary depending on, for example, age, body weight, general health and diet of the subject, or the condition being treated, or the condition of the newborn and his/her physical condition.Other factors to take into account may be according to the mode and time of administration, excretion rate or drug combination.

It is routine practice for the skilled person in the art to asses the therapeutically effective amount to be administered to the subject depending on the variables cited. In a preferred embodiment, *L. salivarius* and B. *longum,* or their preferred embodiments previously mentioned, may each be present in an amount equivalent to between 10⁶ and 10¹² cfu/g of dry composition. The quantity of bacteria which the composition contains is expressed in terms of the equivalent colony forming units of bacteria irrespective of whether they are, all or partly, live, inactivated, dead or fragmented. In a more preferred embodiment, *L. salivarius* is present in the probiotic composition of the invention at an amount of 10⁹ cfu/g of dry composition. In another preferred embodiment, *B. longum* is present in the probiotic composition of the invention at an amount of 10⁹ cfu/g of dry composition.

In the present invention, the therapeutically effective dose can be defined as a daily dose of the probiotic composition which delivers an amount of 10⁶ to 10¹² cfu/g of dry composition of each of the bacteria species that comprise the composition. The composition of the invention may be administered in a single daily dose or multiple doses per day, such as at least 2 doses per day, at least 3 doses per day, at least 4 doses per day. Preferably, the composition of the invention is administered more than once per day. In a preferred embodiment, the composition according to the invention is administered in 2 or 3 doses per day, most preferably in 3 doses per day.

According to the conventional techniques known to those skilled in the art, the composition of the invention may be formulated in combination with an excipient and/or a carrier. Thus, in a preferred embodiment, the composition of the invention further comprises an excipient and/or a carrier.

The term "excipient" refers to a substance which helps to absorb any of the components of the composition of the invention, stabilizes said components or helps in the preparation of the composition in the sense of giving it consistency or providing flavours which make them more pleasant. Thus, excipients could have the function of keeping the components bound together, such as for example starches, sugars or celluloses, a sweetening function, a colorant function, the function of protecting the active ingredient, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material which, included in the galenic forms, is added to the active ingredients or to its associations to enable its preparation and stability, modify its organoleptic properties or determine the physical/chemical properties of the composition and its bioavailability. The "excipient" should allow for the activity of the compounds of the composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerine, amongst others.

The term "carrier" as used herein refers to a substance used in the composition to dilute any of its constituent parts to a certain volume or weight. The carrier is a substance that is inert or has an identical action to any of the elements included in the pharmaceutical composition of the invention. The function of the carrier is to facilitate the incorporation of other elements, to allow a better dosage and administration or to give consistency and form to the composition. Therefore, the carrier is a substance which is used to dilute any of the components of the composition of the present invention to a determined volume or weight, or even without diluting said components, capable of allowing better dosing and administration or giving consistency and form to the composition. In the context of the present invention, the terms "carrier" and "vehicle" are equivalent and can be used interchangeably. Some examples of substances which can serve as carriers are sugars, such as lactose, glucose and sucrose; starches such as corn starch and potato starch; cellulose and its derivatives such as sodium carboxymethycellulose, ethylcellulose and cellulose acetates; powdered tragancanth gum; malt; gelatin; talc; stearic acids; magnesium stearate; calcium sulfate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; pyrogen-free water; isotonic saline; and phosphate buffer solution; skim milk powder; as well as other non-toxic compatible substances used in formulations. Wetting agents and lubricants such as sodium lauryl sulfate, as well as coloring agents, flavoring agents, lubricants, excipients, tableting agents, stabilizers, anti-oxidants and preservatives, can also be present.

The probiotic composition of the invention may be presented in different conformations which may depend on its use or requirements of administration. Therefore, in another preferred embodiment of the use of the invention, the composition is a nutritional composition or a pharmaceutical composition.

In the present invention, the term "nutritional composition" refers to that food, which regardless of providing nutrients to the subject who consumes it, beneficially affects one or more functions of the body, so as to provide better health and wellness. In the present invention, said nutritional composition is intended to the reduction of pathogenic strains in the gastrointestinal tract, the reduction of proinflammatory response, the treatment and/or prevention of necrotic enterocolitis, and/or the treatment and/or prevention of allergies in a subject.

In more preferred embodiment, the nutritional composition is a food. As used herein, the terms "food" and "feed product" are equivalent and can be used indistinctly throughout the present description. The food product can be in liquid, powdery, or solid form. The food may be for human or non-human animal consumption, including infant nutrition and feed. Foods comprise functional foods commonly known in the art. Examples of foods include, but not limited to, feed, dairy products, vegetable products, meat products, snacks, chocolates, beverages, baby food, cereals, fried foods, industrial bakery products and biscuits. Examples of milk products include, but are not limited to, products derived from fermented milk (for example, but not limited to, yogurt or cheese) or non-fermented milk (for example, but not limited to, ice cream, butter, margarine or whey). The vegetable product is, for example, but not limited to, a cereal in any form of presentation, fermented (for example, soy yogurt, oat yogurt, etc.) or unfermented, and a snack. Examples of beverages include, without limitation, juice beverages (e.g., beverages comprising one or more fruit juices and/or one or more vegetable juices), hydration beverages such as those with added electrolytes, frozen or chilled beverages, non-carbonated beverages, zero to low calorie drinks (for example, 0 - 150 kcals and up to 10 grams sugar), such as diet or other reduced calorie beverages, and syrups or concentrates. Non-limiting examples of suitable dairy-containing beverages include milk (e.g., 2% milk) and other beverages containing milk (e.g., coffee drinks containing milk). In some preferred embodiments, the food product is a beverage that may require refrigeration. In some preferred embodiments, the food product is a beverage that is stable and safe to store at ambient conditions not requiring refrigeration. In some embodiments, the food product comprises a low pH of less than about 4.5. Suitable beverages may comprise sweetener, water, dairy, caffeine, carbonation, fruit juice, vegetable juice, food grade acid, or a mixture thereof. Beverage products disclosed herein include ready-to-drink liquid formulations (i.e., ready-to-drink beverages), beverage concentrates, beverage pods, and the like. The term "ready-to-drink" refers to a beverage formulated to be ingested as-is. Thus, in some preferred embodiments, the ready-to-drink beverage requires no dilution or additions prior to ingestion by a consumer. Thus, in a preferred embodiment of the use of the invention, alone or in combination with the rest of preferred embodiments of the present invention, the food product or food is selected from the group consisting of fruit or vegetable juices, ice cream, infant formula, milk, yogurt, cheese, fermented milk, powdered milk, cereals, baked goods, milk-based products, meat products and beverages.

Alternatively to the nutricional composition, the probiotic composition of the invention may be formulated as a pharmaceutical composition. In this case, the excipient or the carrier of the composition is a "pharmaceutically acceptable excipient" or a "pharmaceutically acceptable carrier". This means that the carrier or the excipient should allow for the activity of the compounds of the pharmaceutical composition (herein the *L. salivarius* and B. *longum* bacteria, or their preferred embodiments previously mentioned), that is to say, for it to be compatible with said components. When the form of presentation is liquid, the pharmaceutically acceptable carrier is the diluent. Likewise, a composition, including its components, is said to be "pharmacologically acceptable" if its administration can be tolerated by a recipient subject.

The composition of the invention may further contain prebiotics. Prebiotics may support the growth of probiotics before they are rendered non-replicating. "Prebiotic" means non-digestible food substances that promote the growth of health beneficial micro-organisms and/or probiotics in the intestines. They are not broken down in the stomach and/or upper intestine or absorbed in the GI tract of the person ingesting them, but they are fermented by the gastrointestinal microbiota and/or by probiotics. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructooligosaccharides, galacto-oligosaccharides, isomalto-oligosaccharides, xylooligosaccharides, arabino-xylo oligosaccharides, mannan-oligosaccharides, oligosaccharides of soy, glycosylsucrose, lactosucrose, lactulose, palatinose-oligosaccharides, malto-oligosaccharides, gums and/or hydrolysates thereof, pectins and/or hydrolysates thereof.

Additionally, the probiotic composition of the invention may further comprise other microorganisms or probiotic bacteria in addition to the *L. salivarius* and B. *longum* bacteria, or their preferred embodiments previously mentioned, as well as other components which find use in the probiotic composition such as vitamins and minerals. Thus, in a preferred embodiment, the probiotic composition of the invention further comprises other microorganisms or probiotic bacteria different from *L. salivarius* and *B. longum* bacteria .

Examples of probiotic microorganisms that can be used in addition to *L. salivarius* and *B. longum* in the probiotic composition of the invention are: yeast, such as *Saccharomyces, Debaromyces, Candida, Pichia,* and *Torulopsis;* and bacteria, such as *Bifidobacterium,* propionic acid *Propionibacterium, Streptococcus, Lactococcus, Bacillus,* tablets *Pediococcus, Leuconostoc, Weissella, Oenococcus,* and *Lactobacillus .*

Specific examples of suitable probiotic microorganisms are: *Saccharomyces cereviseae, Bacillus coagulans, Bacillus licheniformis, Bacillus subtilis, Bifidobacterium bifidum, Bifidobacterium infantis, Bifidobacterium longum, Lactobacillus acidophilus, Lactobacillus alimentarius, Lactobacillus casei* subsp. *casei, Lactobacillus casei, Lactobacillus curvatus, Lactobacillus delbruckii* subsp. *lactis, Lactobacillus farciminis, Lactobacillus gasseri, Lactobacillus helveticus, Lactobacillus johnsonii, Lactobacillus rhamnosus, Lactobacillus sakei (Lactobacillus sake), Lactococcus lactis, Pediococcus acidilactici, Pediococcus pentosaceus,* and *Streptococcus thermophilus.*

Examples of vitamins and minerals and other nutrients for use in the probiotic composition of the invention include vitamin A, vitamin B1, vitamin B2, vitamin B6, vitamin B12, vitamin E, vitamin K, vitamin C, vitamin D, folic acid, inositol, Niacin, biotin, pantothenic acid, choline, calcium, phosphorus, iodine, iron, magnesium, copper, zinc, manganese, chlorine, potassium, sodium, selenium, chromium, molybdenum, taurine and L-carnitine.

The present invention relates to a probiotic composition useful in the reduction of pathogenic strains in the gastrointestinal tract, the reduction of proinflammatory response, the treatment and/or prevention of necrotic enterocolitis, and/or the treatment and/or prevention of allergies in a subject.

The term "pathogenic strain" as used herein refers to any microorganism that has the ability to cause disease or whose presence is detrimental to the host. Pathogen microorganisms may include, but are not limited to, *Clostridium, Cronobacter, Escherichia, Klebsiella, Salmonella,* Serratia, *Shigella, Campylobacter, Pseudomonas, Streptococcus, Enterococcus, Staphylococcus,* other Gram-positive and Gram-negative genera, and other species including yeasts, viruses and protozoa. More specifically, pathogenic microorganisms may include, without limitation, *Staphylococcus aureus, Staphylococcus epidermidis, Enterococcus faecalis, Listeria monocytogenes, Streptococcus pyogenes, Streptococcus disgalactiae, Cronobacter sakazakii, Streptococcus agalactiae, Klebsiella pneumoniae, Klebsiella oxytoca, Serratia marcescens.*

Therefore, the expression "reduction of pathogenic strains in the gastrointestinal tract" as used herein refers to the lowering of the number, concentration or volume of pathogenic microorganisms in the gastrointestinal tract of the host in comparison to the number, concentration or volume of pathogenic microorganisms present in subjects which have not been administrated the probiotic composition of the invention. The term "gastrointestinal tract" as used herein refers to the tract from the mouth to the anus which includes all the organs of the digestive system in humans.

The term "proinflammatory response" as used herein refers to the response by the body to disease and/or injury, a response characterized by the classical signs of pain, heat (localized warmth), redness, and swelling. Innumerable insults, as bacterial infection, can trigger an inflammatory response and dispatch inflammatory agents to the site to repair the damage. The term "proinflammatory agent" refers to any of the various agents known to promote inflammation that are produced and/or released by macrophages, including, but not limited to, immunoglobulins IgM and IgA, interleukins, specifically interleukin-6 (IL-6) and interleukin-1β (IL-1β), and tumor necrosis factor alpha (TNF-α), T-helper (Th) 1 response factors like Interferon gamma (INF-y), interleukin 2, 12 and 8, Th2 response factors like interleukin 4, 5 and 13 and RANTES and Th17 response factors like interleukin 9, 17A and 22. Inflammation is a key part of the body's defense system, an indispensable protective response by the body's system of self-defense. Paradoxically, the inflammatory process itself may cause tissue damage while it is engaged in healing and repair. Therefore, another use of the probiotic composition of the invention is the "reduction of proinflammatory response", which, as used herein refers to the decrease in number, concentration or volume of the proinflammatory agents that respond to insults to the gastrointestinal tract, as for example enteral feeding, said reduction being in relation to the number, concentration or volume of the proinflammatory agents in subjects that have not been administrated the probiotic composition of the invention.

As previously mentioned, in infants, especially preterm and LBW infants, due to the poor development of the vesicular system in the bowel, inflammation can occur simply due to enteral feeding which can be too aggressive to the bowel lining. Therefore, the probiotic composition of the invention also finds use in the "treatment and/or prevention of necrotic enterocolitis", since the colonization of the bowel with microorganisms that favor a healthy development of the bowel lining is important in the treatment and/or prevention of necrotic enterocolitis. The term "necrotic enterocolitis" or its abbreviation "NEC" as used herein refer to a medical condition where a portion of the bowel dies. The term "bowel" refers to the alimentary canal below the stomach, the intestine, and is part of the intestinal tract.

Since a main source of allergy development in newborns is enteral feeding, and such allergies can lead to inflammation and subsequently necrotic enterocolitis, the probiotic composition also finds use in the "treatment and/or prevention of allergies". The term "allergy" as used herein refers to a hypersensitivity reaction of the immune system to substances that are normally tolerated. Allergies may be allergic reactions detected by a physician. Once more, the colonization of the bowel with microorganisms that favor a healthy development of the bowel lining and strengthen its resistance to foreign insults leads to the recovery from allergic reactions and to the avoidance of such allergic reactions occurring.

The term "treatment" as used herein refers to fighting the effects caused as a consequence of the disease or pathological condition of interest (like necrotic enterocolitis) in a subject (preferably a human, more preferably an infant), including:
i) Inhibiting the disease or pathological condition, i.e., stopping its development;
ii) Alleviating the disease or pathological condition, i.e. causing the regression of the disease or pathological condition or its symptoms;
iii) Stabilizing the disease or pathological state.

The term "prevention", as herein refers to the avoiding the appearance or reappearance of the condition, that is, avoiding the pathological state of the disease in a subject (preferably a human, more preferably an infant), in particular, when said subject has a predisposition to the pathological condition but has not yet been diagnosed.

The use of the invention is accomplished by administrating the probiotic composition of the invention to a subject, preferably an infant, more preferably a preterm infant and/or low birth weight infant.

The term "subject" as used herein is equivalent to the term "individual"; so both terms can be used interchangeably in the present description. "Subject" means, in addition to any individual, any animal belonging to any species. However, in a preferred embodiment, the subject is a mammal, preferably the mammal is a human being of any race, sex or age. In a more preferredembodiment, the subject is an infant, even more preferably a preterm infant and/or low birth weight infant.

The term "infant" as used herein refers to a child under 12 months of age, such as a child between 0 to 6 months. The term "preterm infant" (or "premature infant") refers to an infant born at less than 37 weeks of gestational age. The term "low birth weight infant" refers to an infant or a preterm infant born with 1500 g or less of weight.

The term "gestational age" or "gestation" as used herein refers to the age of an embryo or fetus (or newborn infant). In humans, a common method of calculating gestational age starts counting either from the first day of the woman's last menstrual period or from 14 days before conception (fertilization). Gestational age is normally presented by the number of weeks since starting counting followed by a plus sign (+) and the number of days that have passed in the current week (e.g. 26 + 5 means a fetus that has an age of 26 weeks and five days). In a preferred embodiment of the use of the invention the preterm infant is born between 28 weeks + 0 days and 30 weeks + 6 days of gestational age.

The composition of the invention for the used of the present invention can be administered at any moment after being born, even within days of the birth of the newborn. In preferred embodiment of the use of the invention, the probiotic composition of the invention is the first administrated within 5 days or less after birth.

The probiotic composition of the invention can be administrated to the subject orally in the form of freeze-dried preparation, paste, liquid, gel, suspension. Dispersions can also be prepared, for example, in glycerol, liquid polyethylene glycols, and mixtures thereof, and in oils.

The probiotic composition of the invention is preferably an enteral composition, i.e., enteral administrated, such as orally. The term "enteral" as used herein refers to the delivery directly into the gastrointestinal tract of a subject (e.g., orally or via a tube, catheter or stoma), as opposed to a direct delivery into the blood stream (parenteral feeding). In a preferred embodiment of the use of the invention, the probiotic composition is enteral administrated. In another preferred embodiment of the use of the invention the preterm infant tolerates enteral feeding of at least 10 milliliters (mL) per kilogram per day.

In another aspect, the present invention relates to a probiotic composition comprising *Lactobacillus salivarius* CECT9527 and *Bifidobacterium longum* CECT30257. Hereinafter, "second probiotic composition of the invention".

In a preferred embodiment, the second probiotic composition of the invention comprises viable and/or unviable cells of *L. salivarius* CECT9527 and B. *longum* CECT30257.

In another preferred embodiment of the second probiotic composition of the invention, alone or in combination with the above preferred embodiment, *L. salivarius* CECT9527 and B. *longum* CECT30257 are each present in an amount of 10⁶ to 10¹² cfu/g of dry composition.

In another preferred embodiment, alone or in combination with the above preferred embodiments, the second probiotic composition of the invention is a pharmaceutical composition or a nutritional composition.

In another preferred embodiment, alone or in combination with the above preferred embodiments, the probiotic composition further comprises a carrier or excipient.

In another preferred embodiment, alone or in combination with the above preferred embodiments, the probiotic composition further comprises other microorganisms or probiotic bacteria different from *L. salivarius* CECT9527 and B. *longum* CECT30257.

In another preferred embodiment, alone or in combination with the above preferred embodiments, the probiotic composition is enteral administrated.

In another particular embodiment, alone or in combination with the above preferred embodiments, the probiotic composition is first administered within 5 days or less after birth.

Definitions and explanations for the above-mentioned preferred embodiments have been disclosed in the previous aspect of the present invention, being applicable to the present inventive aspect relating to the second probiotic composition of the invention.

### BRIEF DESCRIPTION OF THE DRAWINGS

**Figure 1****.** Inhibitory capacity of *L. salivarius* CECT9527 against Gram-positive (1 to 6) and Gram-negative (7 to 13) microorganisms.
**Figure 2****.** Number of samples with lactobacilli and bifidobacteria.
**Figure 3****.** Percentage of infants with positive counts of lactobacilli in their fecal samples.
**Figure 4****.** Percentage of infants with positive counts of Bifidobacterium in their fecal samples.
**Figure 5****.** Concentration of the inflammatory markers after 1 week of treatment.
**Figure 6****.** Calprotectin levels after 1 week of treatment.
**Figure 7****.** Regulatory cytokines at the end of the treatment.
**Figure 8****.** Change in the immunoglobulins at the end of the treatment.

### Examples

### Example 1 - Isolation of L. salivarius CECT9527 and B. longum CECT30257.

### L. salivarius CECT9527)

*L. salivarius* was isolated from the vagina of a healthy women. A vaginal swab was aseptically collected in a sterile tube and kept at 4°C until delivery to the laboratory. The biological material contained in the vaginal swab was resuspended in 1 mL of peptone water. Dilutions of the sample with peptone water were plated in triplicate onto de Man, Rogosa, and Sharpe (MRS, Oxoid, Basingstoke, UK) supplemented with L-cysteine (0.5 g/L) (MRS-Cys) agar plates, which were incubated anaerobically (85% nitrogen, 10% hydrogen, 5% carbon dioxide) in an anaerobic workstation (MINI-MACS, DW Scientific, Shipley, UK) at 37°C for 48 hours.

The strain was identified at the species level as *L. salivarius* by PCR amplification of a section of a 16S rRNA gene variable region using primers pbl16 (5'-AGAGTTTGATCCTGGCTCAG-3' [SEQ ID NO: 3]) and mbl16 (5'-GGCTGCTGGCACGTAGTTAG-3' [SEQ ID NO: 4]) (Kullen et al. 2000. Journal of Applied Microbiology 89, 511-518). PCR conditions were as follows: 96°C for 30 s, 50°C for 30 s and 72°C for 45 s (35 cycles) and a final extension at 72°C for 4 minutes. Amplified fragments were purified using the NucleoSpin Extract II (Macherey-Nagel Gmb; Düren, Germany) and sequenced using the primers cited above on an ABI 377A automated sequencer (Applied Biosystems, Foster City, USA). The sequences were compared with those deposited in the EMBL database using BLAST algorithm (http://www.ncbi.nlm.nih.gov/BLAST).

The fragment sequenced from *L. salivarius* CECT9527 16S rRNA gene comprises the sequence SEQ ID NO:1, which is reproduced below.

AACGTCATCGATGAACAGTTACTCTCACTCGTGTTCTTCTCTAACAACAGAGTTTTACGATC
CGAAGACCTTCTTCACTCACGCGGCGTTGCTCCATCAGACTTGCGTCCATTGTGGAAGATTC
CCTACTGCTGCCTCCCGTAGGAGTTTGGGCCGTGTCTCAGTCCCAATGTGGCCGATCAACCT
CTCAGTTCGGCTACGTATCATCACCTTGGTAGGCCGTTACCCCACCAACTAGTTAATACGCC
GCGGGTCCATCTAAAAGCGATAGCAGAACCATCTTTCATCTAAGGATCATGCGATCCTTAGA
GATATACGGTATTAGCACCTGTTTCCAAGTGTTATCCCCTTCTTTTAGGCAGGTTACCCACG
TGTTACTCACCCGTCCGCCACTCAACTTCTTACGGTGAATGCAAGCATTCGGTGTAAGAAAG
TTTCGTTCGACTTGCATGTATTAGGCACGCCGCCAGCGTTCGTCCTGAGCCAGGATCA

The identification was confirmed by Matrix Assisted Laser Desorption Ionization-Time of Flight (MALDI-TOF) mass spectrometry using a Vitek-MS^{™} instrument (BioMérieux, Marcy l'Etoile, France) in the facilities of Probisearch (Tres Cantos, Spain). Briefly, a portion of a bacterial colony (~1 µL) was directly spotted onto a MALDI sample plate. Then, it was overlaid with 1 µL of a saturated solution of α-cyano-4-hydroxycinnamic acid in acetonitrile (28%) and allowed to dry at room temperature. A mean spectrum was constructed with at least 50 m/z spectra profiles and used for the identification by comparison with the spectra contained in the Myla database (Biomerieux). Identification was defined as a 99-100% match to the species-specific m/z values in the database.

This strain has been genotyped by pulsed-field gel electrophoresis (PFGE) analyses.

### B. longum CECT30257

*B. longum* was isolated in the frame of a study to evaluate the bacterial diversity of the *Bifidobacterium* group in human milk of healthy women and understand their potential translocation from the gut. Women were enrolled in this study according with the following criteria: (a) healthy women without present or past underlying conditions (including mastitis); (b) normal full-term pregnancy; and (c) absence of infant and/or maternal perinatal problems (including mastitis). Milk samples were aseptically collected in sterile tubes at day 7 after delivery and kept at 4°C until delivery to the laboratory, which happened within the first three hours after collection. The study from which this strain was isolated was approved by the Ethical Committee on Clinical Research of Hospital Clinico (Madrid).

Dilutions of the samples with peptone water were plated in triplicate onto de Man, Rogosa, and Sharpe (MRS, Oxoid, Basingstoke, UK) supplemented with L-cysteine (0.5 g/L) (MRS-Cys) agar plates, which were incubated anaerobically (85% nitrogen, 10% hydrogen, 5% carbon dioxide) in an anaerobic workstation (MINI-MACS, DW Scientific, Shipley, UK) at 37°C for 48 hours.

The isolates were examined by phase-contrast microscopy to determine cell morphology and Gram-staining reaction, and tested for oxidase and catalase activities and, also, for good growth (>10⁸ CFU/ml) in MRS broth incubated at 37°C for up to 24 hours.

The isolated strain from a human milk sample was identified at the species level as *B. lomgum* by PCR amplification of a section of a 16S rRNA gene variable region using primers pbl16 (5'-AGAGTTTGATCCTGGCTCAG-3' [SEQ ID NO: 5]) and mbl16 (5'-GGCTGCTGGCACGTAGTTAG-3' [SEQ ID NO: 6]) (Kullen et al. 2000. Journal of Applied Microbiology 89, 511-518). PCR conditions were as follows: 96 °C for 30 s, 50 °C for 30 s and 72 °C for 45 s (35 cycles) and a final extension at 72 °C for 4 minutes. Amplified fragments were purified using the NucleoSpin Extract II (Macherey-Nagel Gmb; Düren, Germany) and sequenced using the primers cited above on an ABI 377A automated sequencer (Applied Biosystems, Foster City, USA). The sequences were compared with those deposited in the EMBL database using BLAST algorithm (http://www.ncbi.nlm.nih.gov/BLAST).

The fragment sequenced from B. *longum* CECT30257 rRNA gene comprises the sequence SEQ ID NO: 2, which is reproduced below.

AATGGTAACTCCCTCACGCTTGCTCCCCGATAAAAGAGGTTTACAACCCGAAGGCCT
CCATCCCTCACGCGGCGTCGCTGCATCAGGCTTGCGCCCATTGTGCAATATTCCCCA
CTGCTGCCTCCCGTAGGAGTCTGGGCCGTATCTCAGTCCCAATGTGGCCGGTCGCCC
TCTCAGGCCGGCTACCCGTCGAAGCCACGGTGGGCCGTTACCCCGCCGTCAAGCTGA
TAGGACGCGACCCCATCCCATACCGCGAAAGCTTTCCCAGAAGACCATGCGATCAAC
TGGAACATCCGGCATTACCACCCGTTTCCAGGAGCTATTCCGGTGTATGGGGCAGGT
CGGTCACGCATTACTCACCCGTTCGCCACTCTCACCACCAAGCAAGCTTGATGGATC

CCGTTCGACTTGCATGTGTTAAGCACGCCGCCAGCGTTCATCCTGAGCCAGATCCAA
ACTC

The complete genome of the strain B. *longum* CECT30257 has been sequenced and analyzed. This strain has been genotyped by pulsed-field gel electrophoresis (PFGE) analyses.

### Example 2 - Determination of antimicrobial spectrum.

### 2.1. Overlay method for L. salivarius CECT9527.

An overlay method was used to determine the ability of the strain to inhibit the growth of different bacteria involved in cases necrotizing enterocolitis and some bacterial infections, including several strains (n=12) of the following species: *Staphylococcus aureus* CECT86 and CECT59, *Staphylococcus epidermidis* CECT232, *Enterococcus faecalis* CECT481, *Listeria innocua* CECT910, *Streptococcus pyogenes* CECT191, *Streptococcus disgalactiae* CECT926 from the Spanish Type Culture Collection, *Cronobacter sakazakii* LMG2762 (Belgian Coordinated Collections of Microorganisms), *Streptococcus agalactiae* 30, *Enterococcus faecalis* 5613, *Klebsiella oxytoca* mc151 and mc153 strains from Probisearch' collection. The plates overlaid with bacterial indicators were incubated at 37°C for 48 hours and, then, were examined for zones of inhibition around the strain streaks. All experiments assaying inhibitory activity were performed in triplicate.

*L. salivarius* CECT9527 showed a good antimicrobial activity against pathogens or those of clinical interest (Table 1). Firstly, in the direct antimicrobial activity assay the size of the inhibition halos ranged from 43 mm against *Streptococcus disgalactiae* CECT926 to 34.3 mm *Staphylococcus pneumoniae* CECT191 (Table 1).

**Table 1. Antimicrobial activity of L. salivarius CECT9527**

| **Indicator organisms** | **inhibition zone (mm)** |
|---|---|
| *Staphylococcus aureus* CECT86 | 20 |
| *Staphylococcus aureus* CECT59 | 30.7 |
| *Staphylococcus epidermidis* CECT232 | ND |
| *Enterococcus faecalis* CECT481 | 19.7 |
| *Listeria innocua* CECT910 | 27 |
| *Streptococcus pyogenes* CECT 191 | 34.3 |
| *Streptococcus disgalactiae* CECT926 | 43 |
| *Cronobacter sakazakii* LMG2762 | 14 |
| *Streptococcus agalactiae* 30 | 14 |
| *Enterococcus faecalis* 5613 | 8 |
| *Klebsiella oxytoca* mc151 | 20 |
| *Klebsiella oxytoca* mc153 | 22 |

| | |
|---|---|
| ND: not detected | |

### 2.2. Co-culture of L. salivarius CECT9527 and pathogens

The co-cultures were made inoculating 1 mL of the *L. salivarius* CECT9527 strain culture and 1 mL the indicator culture, both at 7 log10 cfu/mL (DO ~ 1) in 20 mL of MRS broth. This mixture was incubated at 37 °C in aerobic condition for 17 hours except for *Morganella morganii* 0149-10 and *Escherichia coli* O146 that were incubated for 6 hours. The initial and final bacterial counts of *L. salivarius* CECT9527 and the pathogens microorganisms were made on MRS plates and BHI agar plates, respectively. These are the Gram-positive microorganisms used in this experiment: *Staphylococcus aureus* CECT86, *Streptococcus pyogenes* CECT190, *Staphylococcus epidermidis* CECT231 from the Spanish Type Culture Collection, *Enterococcus faecalis* 5613, *Streptococcus agalactiae* 30 and 41 and *Streptococcus pyogenes* SO-1 strains from Probisearch collection. In addition, Gram-negative microorganisms were tested: *Cronobacter sakazakii* LMG2762 (Belgian Coordinated Collections of Microorganisms), *Klebsiella oxytoca* mc151 and mc153, *Morganella morganii* 0149-10 and *Escherichia coli* O146 (Probisearch collection).

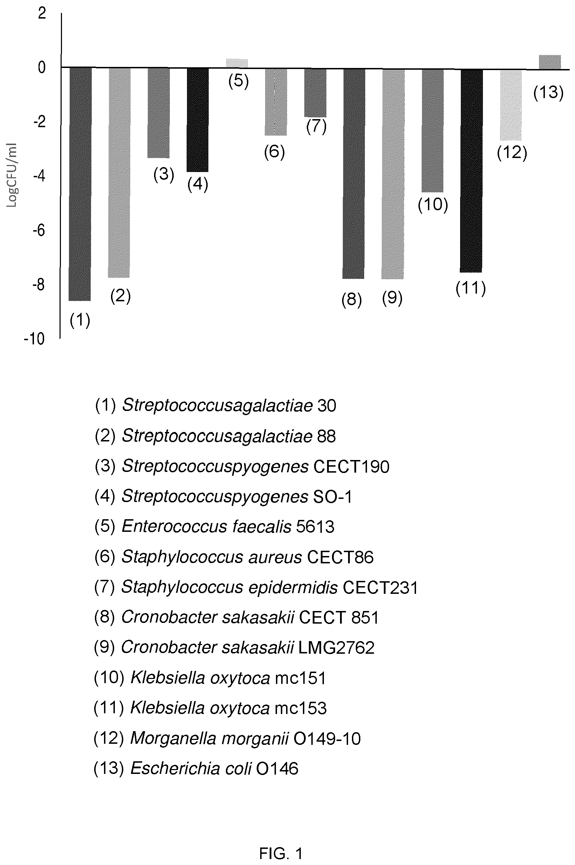

*L. salivarius* CECT9527 was able to inhibit the growth of *St. agalactiae* 30 and St. *agalactiae* 41 up to 6 logarithmic units. It was also effective against *S. epidermidis* CECT231 and *S. aureus* CECT86 with an inhibitory capacity of 2.47 and 1.78 LOG CFU/mL, respectively. *L. salivarius* CECT9527 also showed significant activity against Gram negative microorganisms, with its inhibitory capacity ranging from 2.61 against *Morganella morganii* 0149-10 to 4.53 LOG CFU/mL for *Klebsiella oxytoca* mc151 (Fig. 1).

### 2.3. Overlay method for B. longum CECT30257

An overlay method was used to determine the ability of the strain to inhibit the growth of different bacteria involved in cases necrotizing enterocolitis and some bacterial infections, including several strains (n=5) of the following species: *Staphylococcus aureus* CECT86, *Staphylococcus epidermidis* CECT232, *Klebsiella pneumoniae* CECT144, *Serratia marcescens* CECT977, *Streptococcus pneumoniae* CECT993 and *Escherichia coli* CECT515 from the Spanish Type Culture Collection and *Cronobacter sakazakii* LMG 2762 of Belgian Coordinated Collections of Microorganisms. The plates overlaid with bacterial indicators were incubated at 37°C for 48 hours and, then, were examined for zones of inhibition around the strain streaks. All experiments assaying inhibitory activity were performed in triplicate.

*B. longum* CECT30257 showed a good antimicrobial activity against almost every indicator organism used in this study (Table 2). The size of the inhibition halos ranged from 40 mm against *Serratia marcescens* CECT977 to 24 mm against *Staphylococcus epidermidis* CECT232.

**Table 2. Antimicrobial activity of B. longum CECT30257**

| **Indicator organisms** | **inhibition zone (mm)** |
|---|---|
| *Staphylococcus aureus* CECT86 | 30 |
| *Staphylococcus epidermidis* CECT232 | 24 |
| *Klebsiella pneumoniae* CECT144 | 35 |
| *Serratia marcescens* CECT977 | 40 |
| *Streptococcus pneumoniae* CECT993 | 23.7 |
| *Escherichia coli* CECT515 | 33 |
| *Cronobacter sakazakii* LMG2762 | 26.7 |

### Example 3 - Colonization of intestinal tract of preterm infants

### 3.1. Clinical trial design and criteria

A randomized, double-blind, placebo-controlled study to investigate the effect of the probiotic composition of the invention which comprises *Lactobacillus salivarius* CECT9527 and *Bifidobacterium longum* CECT30257 was conducted in 30 preterm infants from 28 weeks + 0 days to 30 weeks + 6 days of gestation. The inclusion criteria were:
- Preterm infants born between week 28 + 0 days and week 30 + 6 days of gestation.
- Written informed consent signed by the parent or legal guardian.
- Tolerance to enteral feeding, at least 10mL / kg / day.
- Postnatal age ≤ 5 days

And the exclusion criteria:
- Child with malformations.
- With cromosopathies.
- With short bowel syndrome or any surgery in the gastrointestinal tract.
- With defect in the intestinal epithelial barrier.

This study was approved by the "Comité Ético de Investigación Clinica del Hospital Universitario La Paz" on the 24th of May 2018 and registered on clinicaltrials.gov under the number NCT03701906. The study was conducted at the Hospital Universitario La Paz and recruitment was carried out between November 2018 and November 2019.

Participants were randomized assigned to one of the two study groups: the control group with placebo consumption and a probiotic consumption group with a mixture of 1×10⁹ colony forming unit (CFU) of *Lactobacillus salivarius* CECT9527 and 1×10⁸ CFU of *Bifidobacterium longum* CECT30257. The probiotic or the placebo were orally administered daily until discharge from the Neonatal Unit or until the 36th post-gestational week of age. The aim of the study was to investigate the effect of the probiotic composition of the invention in the colonization of the intestinal tract of preterm babies.

Meconium samples were collected prior to probiotic composition or placebo administration and, later, fecal samples were collected weekly for the complete intervention period. Classical microbiological culture techniques and PFGE (Pulse Field Gel Electrophoresis) were used to determine the presence of *L. salivarius* CECT9527 and *B. longum* CECT30257 in fecal samples. Additionally, DNA has been obtained to study the microbiome composition with Illumina technology. The concentration of 18 cytokines, chemokines, growth factors and calprotectin were determined in these samples by using a Luminex 200 system instrument and quantitative ELISA. Growth and clinical morbidities were evaluated as secondary outcomes. Own mother's milk, donor human milk and formula intake were recorded, as type and days of antibiotics.

### 3.1. Results

In this study 30 infants were recruited. Of these, 27 completed the study and 3 subjects withdrew from the study. Both groups were homogeneous regarding demographic characteristics like age at inclusion, gestational age, birth type, gender, ethnicity, birth weight, length and head circumference. Despite that fact, infants from probiotic group suffered a higher number of relevant medical issues during the pregnancy of their mothers. Six of 14 infants of the probiotic group were born after a premature rupture of membranes, while none did it in the placebo group.

The mean number of days of stay at the NICU (neonatal intensive unit care) was 3 days higher in the placebo group than in the probiotic group. Infants from the placebo group suffered 56 Adverse Events versus the 47 of the probiotic group during their participation in the study. Six of 56 were classified as gastrointestinal disorders while 2 of 47 among the infants in the probiotic group.

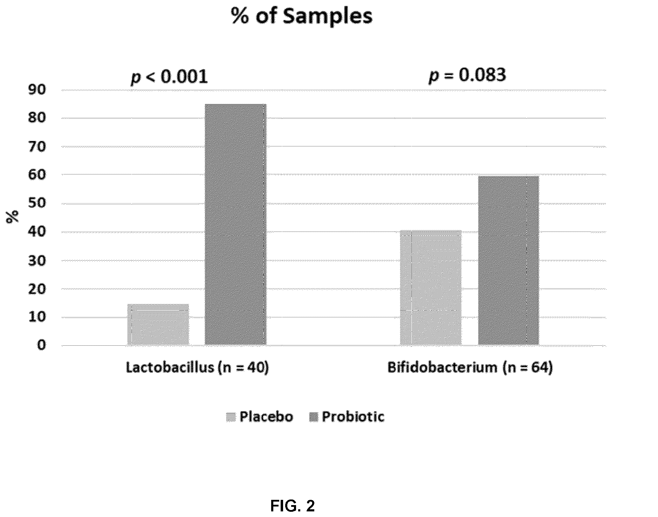

Regarding the microbiological analysis of fecal samples, 170 samples were plated. Among them, *Lactobacillus* and *Bifidobacterium* were isolated from 57 and 30 samples of the probiotic and placebo group respectively (p< 0.001). Bacteria from the Lactobacillus genera were isolated from 34 samples of the probiotic group and in 6 samples of the placebo group (p< 0.001). Additionally, Bifidobacterium was isolated from 38 samples of the probiotic group and 26 of the placebo group (p=0.083) (Figure 2).

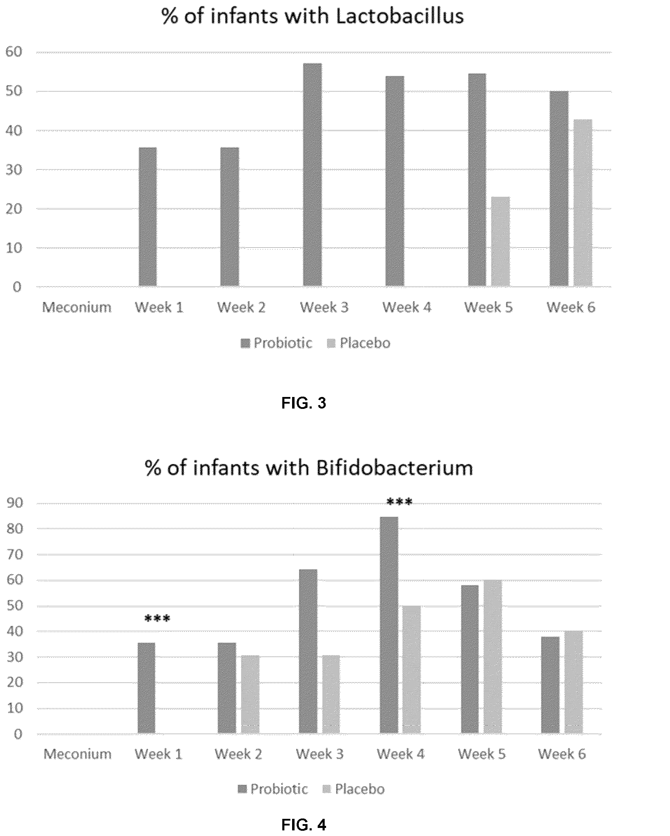

When analyzing the bacterial colonization weekly, the results showed that lactobacillus colonization was delayed in 4 weeks in the placebo group when comparing with the probiotic group. Additionally, the Bifidobacterium colonization was delayed in 1 week in the placebo group (Figures 3 and 4).

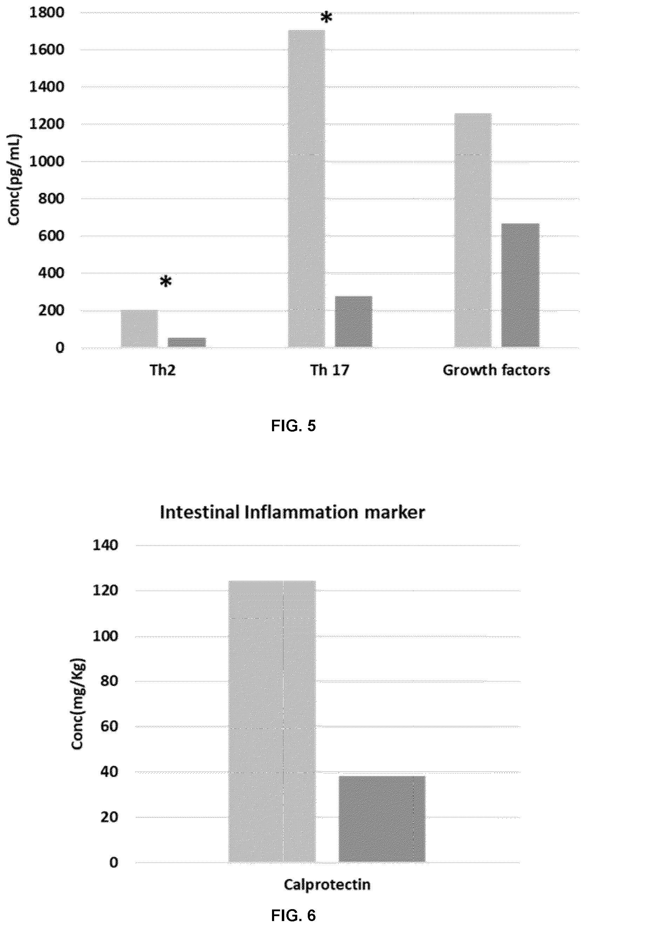

The analysis of the immune parameters showed that inflammation markers were reduced significantly after only one week of treatment in the probiotic group compared with the placebo group (Figures 5 and 6).

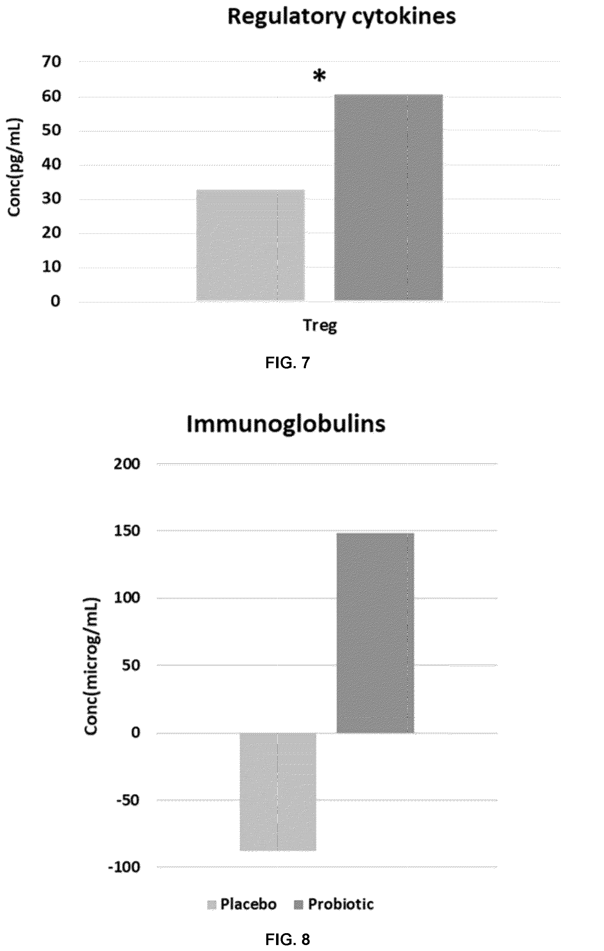

At the end of the treatment the immunomodulatory cytokines and the immunoglobulins detected in samples from the probiotic group were significantly higher than in the placebo group (Figures 7 and 8).

## Claims

1. Probiotic composition comprising *Lactobacillus salivarius* and *Bifidobacterium longum* for use in the reduction of pathogenic microorganisms in the intestinal tract, in the reduction of proinflammatory response, in the treatment and/or prevention of necrotizing enterocolitis, or in the treatment and/or prevention of allergies in a subject, wherein
- the *L. salivarius* strain *is L. salivarius* CECT9527 or a strain derived thereof or a strain having at least 95% of identity with the 16S rRNA sequence of the *L. salivarius* CECT9527, and
- the *B. longum strains is B. longum* CECT30257 or a strain derived thereof or a strain having at least 95% of identity with the 16S rRNA sequence of the B. *longum* CECT30257.

2. Probiotic composition for use according to claim 1, wherein the probiotic composition comprises viable and/or unviable cells of *L. salivarius and B. longum.*

3. Probiotic composition for use according to claim 1 or 2, wherein *L. salivarius* and B. *longum* are each present in an amount of 10⁶ to 10¹² cfu/g of dry composition.

4. Probiotic composition for use according to any one of claims 1 to 4, wherein the composition is pharmaceutical composition or a nutritional composition.

5. Probiotic composition for use according to any one of claims 1 to 4, further comprising a carrier or excipient.

6. Probiotic composition for use according to any one of claims 1 to 5, further comprising other microorganisms or probiotic bacteria different from *L. salivarius* and *B. longum* bacteria.

7. Probiotic composition for use according to any of claims 1 to 6, wherein the subject is an infant, preferably a preterm infant and/or low birth weight infant.

8. Probiotic composition for use according to claim 7, wherein the preterm infant is born between 28 weeks + 0 days and 30 weeks + 6 days of gestational age.

9. Probiotic composition for use according to any one of claims 1 to 9, wherein the composition is enteral administrated.

10. Probiotic composition for use according to any one of claims 1 to 9, wherein the composition is first administered within 5 days or less after birth.

11. Probiotic composition comprising *Lactobacillus salivarius* CECT9527 and *Bifidobacterium longum* CECT30257.

12. Probiotic composition according to claim 11, further comprising a carrier or excipient.

13. Probiotic composition according to claim 11 or 12, wherein *L. salivarius* and B. *longum* are each present in an amount of 10⁶ to 10¹² cfu/g of dry composition.

## Patentansprüche

1. Probiotische Zusammensetzung, umfassend *Lactobacillus salivarius* und *Bifidobacterium longum* zur Verwendung bei der Reduktion pathogener Mikroorganismen im Darmtrakt, bei der Reduktion der proinflammatorischen Reaktion, bei der Behandlung und/oder Prävention von nekrotisierender Enterokolitis oder bei der Behandlung und/oder Prävention von Allergien bei einem Individuum, wobei
- der *L. salivarius-Stamm L. salivarius* CECT9527 oder ein davon abgeleiteter Stamm oder ein Stamm ist, der mindestens 95 % Identität mit der 16S-rRNA-Sequenz von *L. salivarius* CECT9527 aufweist, und
- der *B. longum-Stamm B. longum* CECT30257 oder ein davon abgeleiteter Stamm oder ein Stamm ist, der mindestens 95 % Identität mit der 16S-rRNA-Sequenz von *B. longum* CECT30257 aufweist.

2. Probiotische Zusammensetzung zur Verwendung nach Anspruch 1, wobei die probiotische Zusammensetzung lebensfähige und/oder nicht lebensfähige Zellen von *L. salivarius und B. longum* umfasst.

3. Probiotische Zusammensetzung zur Verwendung nach Anspruch 1 oder 2, wobei *L. salivarius* und *B. longum* jeweils in einer Menge von 10⁶ bis 10¹² koloniebildenden Einheiten (cfu)/g der trockenen Zusammensetzung vorhanden sind.

4. Probiotische Zusammensetzung zur Verwendung nach einem der Ansprüche 1 bis 4, wobei die Zusammensetzung eine pharmazeutische Zusammensetzung oder eine Nährstoffzusammensetzung ist.

5. Probiotische Zusammensetzung zur Verwendung nach einem der Ansprüche 1 bis 4, ferner umfassend einen Träger oder Hilfsstoff.

6. Probiotische Zusammensetzung zur Verwendung nach einem der Ansprüche 1 bis 5, ferner umfassend andere Mikroorganismen oder probiotische Bakterien, die sich von *L. salivarius-* und *B. longum-Bakterien* unterscheiden.

7. Probiotische Zusammensetzung zur Verwendung nach einem der Ansprüche 1 bis 6, wobei das Individuum ein Säugling ist, vorzugsweise ein Frühgeborenes und/oder ein Säugling mit niedrigem Geburtsgewicht.

8. Probiotische Zusammensetzung zur Verwendung nach Anspruch 7, wobei das Frühgeborene zwischen 28 Wochen + 0 Tagen und 30 Wochen + 6 Tagen Gestationsalter geboren wird.

9. Probiotische Zusammensetzung zur Verwendung nach einem der Ansprüche 1 bis 9, wobei die Zusammensetzung enteral verabreicht wird.

10. Probiotische Zusammensetzung zur Verwendung nach einem der Ansprüche 1 bis 9, wobei die Zusammensetzung erstmals innerhalb von 5 Tagen oder weniger nach der Geburt verabreicht wird.

11. Probiotische Zusammensetzung, umfassend *Lactobacillus salivarius* CECT9527 und *Bifidobacterium longum* CECT30257.

12. Probiotische Zusammensetzung nach Anspruch 11, ferner umfassend einen Träger oder Hilfsstoff.

13. Probiotische Zusammensetzung nach Anspruch 11 oder 12, wobei *L. salivarius* und *B. longum* jeweils in einer Menge von 10⁶ bis 10¹² cfu/g der trockenen Zusammensetzung vorhanden sind.

## Revendications

1. Composition probiotique comprenant *Lactobacillus salivarius* et *Bifidobacterium longum* destinée à être utilisée dans la réduction des microorganismes pathogènes dans le tractus intestinal, dans la réduction de la réponse pro-inflammatoire, dans le traitement et/ou la prévention de l'entérocolite nécrosante, ou dans le traitement et/ou la prévention d'allergies chez un sujet, dans laquelle
- la souche *L. salivarius est L. salivarius* CECT9527 ou une souche dérivée de celle-ci ou une souche ayant au moins 95 % d'identité avec la séquence d'ARNr 16S de *L. salivarius* CECT9527, et
- la *souche B. longum est B. longum* CECT30257 ou une souche dérivée de celle-ci ou une souche ayant au moins 95 % d'identité avec la séquence d'ARNr 16S de *B. longum* CECT30257.

2. Composition probiotique destinée à être utilisée selon la revendication 1, dans laquelle la composition probiotique comprend des cellules viables et/ou non viables de *L. salivarius et de B. longum.*

3. Composition probiotique destinée à être utilisée selon la revendication 1 ou 2, dans laquelle *L. salivarius* et *B. longum* sont chacune présentes en une quantité de 10⁶ à 10¹² ufc/g de composition sèche.

4. Composition probiotique destinée à être utilisée selon l'une quelconque des revendications 1 à 4, dans laquelle la composition est une composition pharmaceutique ou une composition nutritionnelle.

5. Composition probiotique destinée à être utilisée selon l'une quelconque des revendications 1 à 4, comprenant en outre un véhicule ou un excipient.

6. Composition probiotique destinée à être utilisée selon l'une quelconque des revendications 1 à 5, comprenant en outre d'autres micro-organismes ou bactéries probiotiques différents des bactéries *L. salivarius* et *B. longum*.

7. Composition probiotique destinée à être utilisée selon l'une quelconque des revendications 1 à 6, dans laquelle le sujet est un nourrisson, de préférence un nourrisson prématuré et/ou un nourrisson de faible poids à la naissance.

8. Composition probiotique destinée à être utilisée selon la revendication 7, dans laquelle le nourrisson prématuré est né entre 28 semaines + 0 jour et 30 semaines + 6 jours d'âge gestationnel.

9. Composition probiotique destinée à être utilisée selon l'une quelconque des revendications 1 à 9, dans laquelle la composition est administrée par voie entérale.

10. Composition probiotique destinée à être utilisée selon l'une quelconque des revendications 1 à 9, dans laquelle la composition est d'abord administrée dans les 5 jours ou moins après la naissance.

11. Composition probiotique comprenant *Lactobacillus salivarius* CECT9527 et *Bifidobacterium longum* CECT30257.

12. Composition probiotique selon la revendication 11, comprenant en outre un véhicule ou un excipient.

13. Composition probiotique selon la revendication 11 ou 12, dans laquelle *L. salivarius* et *B. longum* sont chacune présentes en une quantité de 10⁶ à 10¹² ufc/g de composition sèche.
